## Europäisches Patentamt

## European Patent Office

## Office européen des brevets

(11) Publication number: **0 058 473**
**B1**

(12) **EUROPEAN PATENT SPECIFICATION**

(45) Date of publication of patent specification: **04.09.85**

(51) Int. Cl.⁴: **C 07 D 301/19, B 01 J 23/92**

(21) Application number: **82300317.3**

(22) Date of filing: **21.01.82**

(54) Preparation of soluble molybdenum catalysts for epoxidation of olefins.

(30) Priority: **21.01.81 US 227116**

(43) Date of publication of application:
**25.08.82 Bulletin 82/34**

(45) Publication of the grant of the patent:
**04.09.85 Bulletin 85/36**

(84) Designated Contracting States:
**BE DE FR GB IT NL**

(56) References cited:
**US-A-3 434 975**
**US-A-3 573 226**
**US-A-3 819 663**
**US-A-3 931 044**

(73) Proprietor: **ATLANTIC RICHFIELD COMPANY**
**Arco Plaza 515 S. Flower Street**
**Los Angeles California 90071 (US)**

(72) Inventor: **Mocella, Michael Thomas**
**308 Baywood Road**
**West Chester Pennsylvania 19380 (US)**

(74) Representative: **Cropp, John Anthony David**
**et al**
**MATHYS & SQUIRE 10 Fleet Street**
**London, EC4Y 1AY (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

Oxirane compounds such as ethylene oxide, propylene oxide, and their higher homologs are valuable articles of commerce. One of the most attractive processes for synthesis of those oxirane compounds is described by Kollar in United States Patent No. 3,351,635. According to Kollar, the oxirane compound (e.g. propylene oxide) may be prepared by epoxidation of an olefinically unsaturated compound (e.g., propylene) by use of an organic hydroperoxide and a suitable catalyst such as molybdenum.

During the epoxidation reaction the hydroperoxide is converted almost quantitatively to the corresponding alcohol. That alcohol may be recovered as a coproduct with the oxirane compound. However, it is the oxirane which is of primary concern.

Kollar teaches that oxirane compounds may be prepared from a wide variety of olefins. Lower olefins having three or four carbon atoms in an aliphatic chain are advantageously epoxidized by the process. The class of olefins commonly termed alpha olefins or primary olefins are epoxidized in a particularly efficient manner by the process. It is known to those in the art that primary olefins, e.g., propylene, butene-1, decene-1 hexadecene-1 etc., are much more difficultly epoxidized than other forms of olefins, excluding only ethylene. Other forms of olefins which are much more easily epoxidized are substituted olefins, alkenes with internal unsaturation, cycloalkenes and the like. Kollar teaches that notwithstanding the relative difficulty in epoxidizing primary olefins, epoxidation proceeds more efficiently when molybdenum, titanium or tungsten catalysts are used. Molybdenum is of special interest. Kollar teaches that activity of those metals for epoxidation of primary olefins is surprisingly high and can lead to high selectivity of propylene to propylene oxide. These high selectivities are obtained at high conversions of hydroperoxide (50% or higher) which conversion levels are important for commercial utilization of the technology.

Kollar's epoxidation reaction proceeds under pressure in the liquid state and, accordingly, a liquid solution of the metal catalyst is preferred. Preparation of a suitable catalyst is taught by Sheng et al in United States Patent No. 3,434,975. According to Sheng, the reaction-medium soluble epoxidation catalyst may be prepared by reacting molybdenum metal with an organic hydroperoxide, peracid or hydrogen peroxide in the presence of a saturated alcohol having one to four carbon atoms.

Another molybdenum epoxidation catalyst is described by Bonetti et al in United States Patent No. 3,480,563. Bonetti teaches that molybdenum trioxide may be reacted with a primary saturated acyclic alcohol having 4 to 22 carbon atoms or with a mono- or polyalkylene glycol monoalkyl ether. The reaction involves heating the molybdenum trioxide in the alcohol or ether to produce an organic soluble molybdenum catalyst.

Maurin et al in United States Patent No. 3,822,321 describes oxidizing olefins with a hydroperoxide using a molybdenum catalyst prepared by reacting a molybdenum compound such as molybdic acid of a molybdic salt with a polyalcohol.

A molybdenum catalyzed epoxidation of olefins is described by Lines et al in United States Patent No. 4,157,346. The catalyst is prepared by reacting an oxygen containing molybdenum compound with an amine (or an amine N-oxide) and alkylene glycol.

In United States Patent No. 3,887,361 Lemke discloses that spent catalyst solutions obtained from the process of epoxidation of olefins with hydroperoxides in the presence of molybdenum may be treated to precipitate and separate dissolved molybdenum. The Lemke process involves mixing the spent catalyst solution with 5 to 50 per cent by weight of tertiary-butyl alcohol and heating the mixture to 100 to 300°C in a closed vessel, preferably under a pressure of from 5 to 50 bars for about 2 to 3 hours, or under reflux. The thermal treatment can be carried out in the presence of such reducing gases as hydrogen or carbon monoxide or inert gases such as nitrogen or carbon dioxide. That procedure results in precipitation of molybdenum as a finely divided solid which is then separated. The solid is disclosed to be suitable for recycle as such into further epoxidation reactions. Also, the solid may be dissolved in organic acids such as those obtained in the "Oxo process" for the production of oxygenated organic derivatives. The Lemke solids typically contain about 30 to 40% by weight of molybdenum.

It has now been discovered that spent soluble molybdenum catalyst can be effectively regenerated and reused in the epoxidation of olefins thereby making it possible to continuously regenerate, recycle and reuse molybdenum catalyst in a continuous process for molybdenum catalyzed epoxidation of olefins with a hydroperoxide.

According to the present invention, there is provided a process for the epoxidation of an olefinic compound with an organic hydroperoxide in the presence of a molbydenum epoxidation catalyst including the steps of treating the epoxidation reaction product mixture to remove unreacted olefin, alkylene oxide product and by-product alcohol to leave a spent catalyst solution containing essentially all the molybdenum values employed in the epoxidation, precipitating a molybdenum-containing solid from said solution and redissolving the molybdenum in said solid characterised in that (i) the molybdenum-containing solid is precipitated by heating said solution in the presence of tertiary butyl alcohol or water and the solid is separated, (ii) a solubilized molybdenum-containing composition capable of being employed as catalyst for the epoxidation is obtained therefrom by contacting the solid with a mixture comprising a monohydroxy alcohol, a polyhydroxy alcohol and an organic peroxide or hydroperoxide, and (iii) a catalytic amount of said composition is recycled to the epoxidation as catalyst therefor.

The term "spent catalyst solution" is used herein to mean that fraction of the epoxidation reaction product effluent remaining after removal of unreacted olefin (for example, propylene), alkylene oxide (for example, propylene oxide) and a major portion of the alcohol corresponding to the hydroperoxide (for example, tertiary butyl hydroperoxide) used in the epoxidation reaction. Spent catalyst solution, apart from molybdenum compounds, contains some alcohol, acids and other low molecular weight oxygenated compounds and said spent catalyst solution is generally not subjected to any chemical treatment before being subjected to the process of the present invention. The spent catalyst solution may, for example, comprise the distillation bottoms treated in British Patent Specification 1,317,480 or the liquid residue obtained from the wiped film evaporation process according to Levine U.S. Patent 3,819,663, and said spent catalyst solution can contain molybdenum at levels of up to about 5% by weight.

Solid precipitates of molybdenum-containing compounds are obtained from spent catalyst solutions by a variety of methods. The present invention relates to a process for dissolving those solids to produce an active soluble epoxidation catalyst for use in processes such as those taught by Kollar.

Accordingly, this invention avoids the necessity of disposal of spent molybdenum catalyst solutions which is detrimental from both economic and ecological view points. These and other objects of the invention will become apparent from the following description and examples.

The molybdenum catalyzed hydroperoxide epoxidation crude reaction product from processes such as Kollar, discussed above, can be purified by distilling off unreacted olefin, epoxide and alcohol corresponding to the hydroperoxide. The remaining residue contains high boiling by-products and dissolved molybdenum catalyst. If the residue is recycled directly into further epoxidations, the yield of epoxide is adversely effected. Accordingly, to reuse the molybdenum it must be separated from the polymeric by-products and regenerated. One method of achieving the separation of the molybdenum from the distillation residue in accordance with the present invention, is by thermal precipitation in the presence of tertiary butyl alcohol according to the teachings of Lemke, noted above.

Another method of achieving the separation is by the thermal precipitation procedure disclosed and claimed in our copending European patent application No. 82300318.1 published as 0056740 wherein the spent catalyst solution is mixed with water in an amount sufficient to produce a two-phase system comprising an organic phase and an aqueous phase which is rich in the molybdenum and heating the aqueous phase whereby to precipitate a molybdenum-containing solid.

The heat treatment, which preferably involves heating to a temperature of 120°C to 225°C, especially about 200°C, for a period of from 15 minutes to 3 hours, can be effected after separation of the aqueous phase from the organic phase or while the phases are in contact. The water is preferably in an amount of from 0.25 to 4 parts, and most preferably about 1 part, per part of spent catalyst solution.

In general, the thermal precipitation of the molybdenum-containing solid involves heating the residue containing dissolved molybdenum in the presence of tertiary butyl alcohol or water or precipitate a solid. The solid precipitate typically contains about 30—40% molybdenum, by weight.

Reuse of molybdenum in the solid precipitate requires an efficient method to solubilize the molybdenum so that a high quality epoxidation can be obtained. In accordance with the present invention, an active catalyst solution is obtained by contacting the solid thermal precipitate with an admixture of a monohydroxy alcohol, a poly-hydroxy alcohol and an organic peroxide or hydroperoxide.

Suitable mono- and poly-hydroxy alcohols include aliphatic and aromatic alcohols and poly-hydroxy alcohols such as glycols and alkylene ethers thereof. Although such compounds may be substituted with functional groups which are inert to the reactants present, e.g. halo such as chloro of fluoro; nitro; cyano; carbonyl; and carboxyl, the readily available aliphatic and aromatic mono- and poly-hydroxy alcohols containing up to about 12 carbon atoms are particularly satisfactory for use in the present invention. Illustrative suitable monohydroxy and poly-hydroxy alcohols are methanol, ethanol, tertiary butyl alcohol, phenol and benzyl alcohol, and polyhydroxy compounds, also containing up to about 12 carbon atoms, such as glycols and derivatives thereof, such as glycol ethers, provided these compounds contain at least one hydroxyl group; typical illustrative polyhydroxy compounds include ethylene glycol, propylene glycol, 1,4-butanediol, catechol and alkylene ethers of such glycols, including the methyl and ethyl ethers thereof.

The temperature employed to solubilize the precipitated molybdenum solids may be in the range of 20°C to 130°C. Temperatures lower than 20°C necessitate unduly long reaction times and are not favored. A particularly convenient temperature is the reflux temperature of the liquid into which solids are being solubilized. Atmospheric pressure for the solubilization reactions is convenient and effective or when the reaction is carried out at higher temperatures which would cause volatilization of the liquid sufficient pressure may be utilized to maintain the liquid phase.

The time required to solubilize the precipitated solids is a function of both temperature and the alcohol species employed. Generally, solubilization requires from 0.5 to 4 hours.

Solubilization has also been observed to be somewhat dependent on the ratio of precipitated solids to solubilizing liquid. Although molybdenum concentrations of up to 5% by weight have been found to be capable of being solubilized by the process of the present invention, preferred amount of solids to be solubilized contain up to about 2 parts, by weight, per 100 parts by weight of solubilizing liquid. As higher levels of solids are present, the stability of the solution obtained is adversely effected. Thus the stability of the solubilized molybdenum depends on both concentration as well as temperature.

It has been surprisingly found that a mixture of monohydroxy alcohol and polyhydroxy alcohol has a synergistic effect on solubilizing precipitated solids in the presence of an organic peroxide or hydroperoxide to produce a regenerated catalyst. Glycol added to alcohol appears to have the effect of stabilizing the molybdenum in solution and prevents reprecipitation. When propylene glycol is used, a minimum of 2 moles of glycol per mole of solubilized molybdenum is generally used. The molar ratio suggests that the stabilizing effect is achieved by complexing the soluble molybdenum with glycols, which bind to more than one metal site, to produce more stable complexes than those formed by monohydroxy compounds. See Basolo & Pearson, "Mechanisms of Inorganic Reactions," John Wiley & Sons, Inc. (1968) pages 223 et seq. Accordingly, when polyhydroxy alcohols having more than two hydroxyl groups are employed, somewhat less than two moles of the alcohol per mole of solubilized molybdenum may be effective because of the higher hydroxyl number. Also, solubilization can be effected in pure glycol (i.e., a large excess). However, large excesses of glycol should not be added to subsequent epoxidations and hence are not favored for molybdenum solubilization.

The third component of the solubilizing liquid is an organic peroxide or hydroperoxide such as tertiary butyl hydroperoxide or ditertiary butyl peroxide and peracids such as peracetic acid. This component may be used in an amount, up to the amount of the total reaction mixture, by weight, but preferably is used in an amount up to about 40%, by weight, of the solubilizing liquid.

It is desirable also to avoid adding excess hydroxyl-containing compound, particularly primary alcohol, to the epoxidation reaction when solubilized regenerated molybdenum catalyst of this invention is employed. Accordingly, after the solubilization of thermally precipitated solids, it is desirable to concentrate the molybdenum in solution particularly if substantial quantities of primary alcohol are used in the dissolution reaction. This is achieved by distilling the solution to remove excess alcohol. By this concentration procedure, solutions containing 5% or more dissolved molybdenum may be obtained. As in the solubilization itself, the presence of polyhydroxy alcohol stabilizes the solution in the concentrating procedure. However, a somewhat higher level of polyhydroxy alcohol is required for this effect. It has been found that mixtures containing at least 6 equivalents of polyhydroxy alcohol per equivalent of solubilized molybdenum may yield homogeneous concentrated solutions upon distillation. Lower levels of polyhydroxy alcohol permit solid separation before completion of the distillation. For solubilization, the desirable amount of polyhydroxy alcohol is more than 10 equivalents per equivalent of solubilized molybdenum. The preferred amount of polyhydroxy alcohol is 10 to 20 equivalents per equivalent of solubilized molybdenum. The concentrated homogeneous solution of molybdenum containing polyhydroxy alcohol as the primary component is suitable for direct addition in catalytic amounts to an epoxidation reaction to supply the necessary molybdenum.

In order to further illustrate the subject matter of the present invention, the following examples are provided. However, it is to be understood that the examples are merely illustrative and are not intended as being restrictive of the invention herein disclosed and as defined by the annexed claims.

Parts and percentages are by weight and temperatures are given in degrees centigrade unless otherwise specified.

Example 1

A spent catalyst stream from a commercial molybdenum catalyzed epoxidation of propylene was thermally treated with tertiary butyl alcohol according to Lemke U.S. Patent 3,887,361 to precipitate dissolved molybdenum as a solid. To regenerate that solid as a reusable soluble molybdenum catalyst, 0.656 parts of the solid was reacted with 23 parts of methanol, 5 parts of a solution of about 40% by weight tertiary butyl hydroperoxide in tertiary butyl alcohol, and 4 parts of propylene glycol by heating the reactants together at 60° for 30 minutes. The reaction mixture was then filtered to remove the trace of solids which remained. The filtered solution was then distilled at a temperature of 110°C to remove methanol and other low boiling materials. The resulting homogeneous solution remaining in the distillation pot had the following composition by weight:

47,500 ppm dissolved Mo
48.0% propylene glycol
42.2% t-butyl alcohol

The concentrated solution was an active catalyst suitable for reuse in epoxidation of propylene.

Example 2 (Comparative)

A thermally precipitated molybdenum-containing solid was obtained according to the Lemke procedure from a spent catalyst solution from a commercial propylene epoxidation as in Example 1. To regenerate that molybdenum-containing solid as a reusable soluble molybdenum catalyst, 0.654 parts of the solid was reacted with 27.01 parts of tertiary butyl alcohol and 5.03 parts of a solution of about 40% by weight tertiary butyl hydroperoxide in tertiary butyl alcohol, by heating the reactants together at 75° for 2 hours. The solution obtained after reaction amounted to 32.5 parts and contained 6450 ppm of dissolved molybdenum. A small amount (0.078 parts) of solid remained undissolved and that solid contained 49.5% by weight molybdenum. Thus, 100% by weight of the original molybdenum charged was recovered and accounted for as solid or solution. Dissolved molybdenum amounted to 84% by weight of that charged.

### Example 3

A thermally precipitated molybdenum-containing solid was obtained from a spent catalyst solution from a commercial propylene epoxidation as in Example 1. To regenerate that molybdenum-containing solid as a reusable soluble molybdenum catalyst, 0.655 parts of the solid was reacted with 27 parts of tertiary butyl alcohol, 5.0 parts of a solution of about 40% by weight tertiary butyl hydroperoxide in tertiary butyl alcohol, and 1.03 parts of propylene glycol by heating the reactants together at 90° for 2 hours. The solution obtained after reaction amounted to 33.56 parts and contained 7580 ppm of dissolved molybdenum. A small amount (0.031 parts) of solid remained undissolved. Dissolved molybdenum amounted to 96% by weight of that charged.

This example shows that a small amount of added glycol along with alcohol results in higher levels of molybdenum solubilization, compared to Example 2.

### Example 4 (Comparative)

A thermally precipitated molybdenum-containing solid was obtained from a spent catalyst solution from a commercial propylene epoxidation as in Example 1. To regenerate that molybdenum-containing solid as a reusable soluble molybdenum catalyst, 0.328 parts of the solid was reacted with 38.12 parts of tertiary-butyl alcohol by heating the reactants together at 90° for one hour. The solution obtained after reaction amounted to 37.98 parts and contained 2470 ppm of dissolved molybdenum. A small amount (0.053 parts) of solid remained undissolved and that solid contained 56.7% by weight molybdenum. Thus 99% by weight of the original molybdenum charged was recovered and accounted for as solid or solution. Dissolved molybdenum amounted to 75% by weight of that charged.

### Example 5

A thermally precipitated molybdenum-containing solid was obtained from a spent catalyst solution from a commercial propylene epoxidation as in Example 1. To regenerate that molybdenum-containing solid as a reusable soluble molybdenum catalyst, 0.654 parts of the solid was reacted with 27 grams of propylene glycol and 5.0 parts of a solution of about 40% by weight tertiary butyl hydroperoxide in tertiary butyl alcohol, by heating the reactants together at 90° for 2 hours. The solution obtained after reaction amounted to 32.10 parts and contained 9160 ppm of dissolved molybdenum. A small amount (0.008 parts) of solid remained undissolved. Dissolved molybdenum amounted to greater than 99% by weight of that charged.

### Example 6

A thermally precipitated molybdenum-containing solid was obtained from a spent catalyst solution from a commercial propylene epoxidation as in Example 1. To regenerate that molybdenum-containing solid as a reusable soluble molybdenum catalyst, 0.654 parts of the solid was reacted with 26 parts of methanol, 5 parts of a solution of about 40% by weight tertiary butyl hydroperoxide in tertiary butyl alcohol, and 1.0 part of propylene glycol by heating the reactants together at 60° for 30 minutes. The solution obtained after reaction amounted to 33.05 parts and contained 8700 ppm of dissolved molybdenum. A small amount (0.005 parts) of solid remained undissolved. Dissolved molybdenum amounted to greater than 99% by weight of that charged.

This example is similar to Example 1; but shows that lower levels of propylene glycol are satisfactory for stabilization of the dissolved molybdenum then required to obtain a homogeneous solution upon concentration.

A stainless steel autoclave is charged with 70 parts propylene and heated to 130°. At that point, is added to the reactor a mixture of 20 parts of tertiary butyl hydroperoxide (TBHP) and 50 parts of tertiary butyl alcohol which contains 100 ppm molybdenum from the solution obtained above. After 60 minutes reaction at 130°, the mixture is quenched and analyzed for propylene oxide (PO) and unreacted tertiary butyl hydroperoxide. The TBHP reacted amounts to 93% of that charged, and PO is produced in 88% selectivity versus hydroperoxide reacted.

**Claims**

1. A process for the epoxidation of an olefinic compound with an organic hydroperoxide in the presence of a molybdenum epoxidation catalyst including the steps of treating the epoxidation reaction product mixture to remove unreacted olefin, alkylene oxide product and by-product alcohol to leave a spent catalyst solution containing essentially all the molybdenum values employed in the epoxidation, precipitating a molybdenum-containing solid from said solution and redissolving the molybdenum in said solid characterised in that (i) the molybdenum-containing solid is precipitated by heating said solution in the presence of tertiary butyl alcohol or water and the solid is separated, (ii) a solubilized molybdenum-containing composition capable of being employed as catalyst for the epoxidation is obtained therefrom by contacting the solid with a mixture comprising a monohydroxy alcohol, a polyhydroxy alcohol and an organic peroxide or hydroperoxide, and (iii) a catalytic amount of said composition is recycled to the epoxidation as catalyst therefor.

2. A process as claimed in Claim 1 which further includes the step of (IV) separating the solubilized molybdenum-containing composition of step (ii) from any remaining solid material priot to recycling it to the epoxidation.

3. A process as claimed in Claim 1 or Claim 2 wherein the polyhydroxy compound is a glycol and is used in an amount of at least two moles per mole of molybdenum to be solubilized.

4. A process as claimed in Claim 1, Claim 2 or Claim 3 wherein step (ii) is effected at a temperature in the range of 20°C to 130°C.

## Revendications

1. Procédé pour l'époxydation d'un composé oléfinique avec un hydroperoxyde organique en présence d'un catalyseur d'époxydation en molybdène, comprenant les étapes de traitement du mélange de produits de réaction d'époxydation pour éliminer l'oléfine n'ayant pas réagi, l'oxyde d'alcoylène produit et l'alcool sous-produit pour laisser une solution de catalyseur usé contenant essentiellement toutes les entités molybdéniques employées dans l'époxydation, de précipitation d'un solide contenant du molybdène à partir de cette solution et de redissolution de molybdène dans ce solide, caractérisé en ce que:

i) on fait précipiter le solide contenant le molybdène en chauffant cette solution en présence d'alcool ter-butylique ou d'eau et on sépare le solide,

ii) on obtient à partir du solide une composition contenant le molybdène solubilisé pouvant être utilisée à titre de catalyseur pour l'époxydation, en mettant ce solide en contact avec un mélange comprenant un alcool monhydroxy, un alcool polyhydroxy et un peroxyde ou hydroperoxyde organique, et

iii) on recycle une quantité catalytique de cette composition vers l'époxydation, en tant que catalyseur pour cette réaction.

2. Procédé suivant la revendication 1, caractérisé en ce qu'il comprend de plus l'étape iv) de séparation de la composition contenant du molybdène solubilisé provenant de l'étape ii) d'un quelconque matériau solide, avant recyclage de celle-ci vers la réaction d'époxydation.

3. Procédé suivant la revendication 1, caractérisé en ce que le composé polyhydroxy est un glycol et qu'il est utilisé en une quantité d'au moins deux moles par mole de molybdène à solubiliser.

4. Procédé suivant la revendication 1, la revendication 2 ou la revendication 3, caractérisé en ce que l'étape ii) est effectuée à une température comprise dans la gamme de 20°C à 130°C.

## Patentansprüche

1. Verfahren zur Epoxidation einer olefinischen Verbindung mit einem organischen Hydroperoxid in Gegenwart eines Molybdän-Epoxidationskatalysators mit den Stufen der Behandlung des Epoxidations-Reaktionsproduktgemisches zur Entfernung von nicht umgesetztem Olefin, Alkylenoxid-Produkt und Nebenprodukt-Alkohol, um eine verbrauchte Katalysatorlösung zurückzulassen, die im wesentlichen die Molybdänwerte enthält, die bei der Epoxidation eingesetzt wurden, Ausfällung eines Molybdän-haltigen Feststoffes aus der Lösung und erneuten Lösens des Molybdäns in dem Feststoff, dadurch gekennzeichnet, daß

(i) der Molybdän-haltige Feststoff durch Erhitzen der Lösung in Gegenwart von tert.-Butylalkohol oder Wasser ausgefält wird und der Feststoff abgetrennt wird,

(ii) eine solubilisierte Molybdän-haltige Zusammensetzung, die als Katalysator für die Epoxidation eingesetzt werden kann, daraus erhalten wird, indem der Feststoff mit einem Gemisch in Berührung gebracht wird, welches einen Monohydroxyalkohol, einen Polyhydroxyalkohol und ein organisches Peroxid oder Hydroperoxid enthält, und

(iii) eine katalytische Menge der Zusammensetzung zur Epoxidation als Katalysator dafür zurückgeführt wird.

2. Verfahren nach Anspruch 1, welches ferner die Stufe (iv) umfaßt, in der die solubilisierte Molybdän-haltige Zusammensetzung aus Stufe (ii) von jeglichem verbleibenden Festmaterial abgetrennt wird, bevor sie zur Epoxidation zurückgeführt wird.

3. Verfahren nach Anspruch 1 oder Anspruch 2, worin die Polyhydroxy-Verbindung ein Glykol ist und in einer Menge von mindestens 2 Molen pro Mol des zu solubilisierenden Molybdäns verwendet wird.

4. Verfahren nach Anspruch 1, 2 oder 3, worin Stufe (ii) bei einer Temperatur im Bereich von 20°C bis 130°C durchgeführt wird.